# EUROPEAN PATENT SPECIFICATION

(11) **EP 2 674 136 B1**
(45) Date of publication and mention of the grant of the patent: **30.03.2016**
(21) Application number: 12172213.6
(22) Date of filing: 15.06.2012
(51) Int. Cl.: A61F 5/01

(54) **Ankle protecting device**
Knöchelschutzvorrichtung
Dispositf de protection de la cheville

(43) Date of publication of application: 18.12.2013
(73) Proprietor: Chen, Tsan-Jee, Taipei City 114 (TW)
(72) Inventor: Chen, Tsan-Jee, Taipei City 114 (TW)
(74) Representative: Wittmann, Günther

(56) References cited:
- US-A1- 2005 085 755
- US-A1- 2009 076 428
- US-A1- 2009 198 165

## Description

### BACKGROUND OF THE INVENTION

### 1. Field of the Invention

The present invention is a human body protecting device, more particularly to an ankle protecting device that provides adequate supports and increases the stability of an ankle.

### 2. Description of the Prior Art

Adequate sports are good to health of human beings, but sports injuries happen as always, and ankle sprain is the highest possibility for sports injuries.

The factors to ankle sprain are mostly as that a body is loosing balance and is stepping on other people's feet or is being tumbled while landing. Local joint swelling, pain, and even causing a fracture are happening while in ankle sprain. After happening, if the treatment is not adequate, tibia, talus within the ankle joint (also known as ankle) and calcaneal may be displaced or stiff tight. Hence, ankle may be in trouble very easily while moving, since the joint is not active enough and easily looses balance thereof.

Prior ankle protecting devices are plentiful, and the most common one is an elastic bandage, wherein a tubular elastic bandage is capable of constricting the ankle, and another stripe bandage can wind around the ankle so as to prevent ankle injuries. Such bandages are easy to construct and provide basic protections and preventions due to the retractility and elasticity for support and oppression. Further that, the winding ways of some elastic bandages are more complex and inconvenient, not suitable for general users.

Another prior ankle protecting device includes a tubular elastic sheath and an elastic piece that are on a lower leg or heel and on the left and right sides of the elastic sheath for constricting an ankle. The angles of internal flipping and outer flipping of the ankle are restricted by the elastic sheath and the elastic piece in order to reduce the possibility of injury. Such ankle protecting device uses the two elastic pieces elongating downward to be over the ankle and the tubular elastic sheath to provide supporting forces. Such elastic piece and sheath are better than aforesaid but only restrict the internal flipping and outer flipping and do not provide suitable restriction and protection for the forward flipping and backward flipping of the sole.

US 2005/0085755 A1 describes a rear entry ankle brace for providing support and stability to an ankle, including a rigid internal support member covered on both sides by a soft durable material. The ankle brace is secured about a leg with an adjustable strap. A flexible heel is provided for facilitating walking

### SUMMARY OF THE INVENTION

The main objective of the present invention is to provide an ankle protecting device that restricts the angles of inner flipping, outer flipping, forward flipping, and backward flipping of the sole, so that the stability of the ankle is increased, the possibility of sprain is decreased, further, the effectiveness of oppression and preventing swelling are achieved as well.

The object of the invention is achieved by a device according to claim 1. Advantageous developments are subject to the dependent claims.

An embodiment of the ankle protecting device of the present invention comprises:
a wrapping sheath, which has retractility and elasticity and has a lower-leg sheath, a foot sheath and at least one loincloth that are integrated with thereof, the loincloth constricts the wrapping sheath on a sole, an ankle and a lower leg and is adjustable for tightness thereof;
an internal supporting plate and an outer supporting plate, which are fastened on the left and right sides of the wrapping sheath respectively, the shapes of the internal supporting plate and the outer supporting plate are symmetrical to each other, the internal supporting plate and the outer supporting plate elongate to be over the ankle along the internal and outer sides of the lower leg and then to the two sides of the sole toward the top of the sole; and
a foot bottom supporting plate, which is fastened on the bottom of the foot sheath, a three-point support is formed by the foot bottom supporting plate, the internal supporting plate and the outer supporting plate in order to support the internal and outer sides of the lower leg, the ankle and the sole, and the angles of inner flipping, outer flipping, forward flipping, and backward flipping of the sole shall be restricted, so that the stability of the ankle is increased, the possibility of sprain is decreased, further, the effectiveness of oppression and preventing swelling are achieved as well.

For a preferred embodiment, both the lower-leg sheath and the foot sheath have three surfaces, the lower-leg sheath has a back surface, a left side surface and a right side surface in order to wrap around the back side, the inner side and the outer side of the lower leg, the foot sheath of the wrapping sheath has a bottom portion, a left side portion and a right side portion in order to wrapped around the bottom portion, the inner side and the outer side of the sole.

For a preferred embodiment, the foot sheath has a bottom portion, a left side portion, a right side portion, and a top surface portion connecting the left side portion and the right side portion in order to wrapped around the bottom portion, the back portion, the inner side and the outer side of the sole.

For a preferred embodiment, the internal supporting plate, the outer supporting plate and the foot bottom supporting plate are made by aluminum or plastic.

According to above descriptions, the ankle protecting device is able to constrict the wrapping sheath on the sole, the ankle and the lower leg via the loincloth and is adjustable for tightness thereof. A three-point support is formed by the foot bottom supporting plate, the internal supporting plate and the outer supporting plate in order to support the internal and outer sides of the lower leg, the ankle and the sole, and the angles of inner flipping, outer flipping, forward flipping, and backward flipping of the sole shall be restricted, so that the stability of the ankle is increased, the possibility of sprain is decreased, further, the effectiveness of oppression and preventing swelling are achieved as well.

Other and further features, advantages, and benefits of the invention will become apparent in the following description taken in conjunction with the following drawings. It is to be understood that the foregoing general description and following detailed description are exemplary and explanatory. The accompanying drawings are incorporated in and constitute a part of this application and, together with the description, serve to explain the principles of the invention in general terms. Like numerals refer to like parts throughout the disclosure.

### BRIEF DESCRIPTION OF THE DRAWINGS

The objects, spirits, and advantages of the preferred embodiments of the present invention will be readily understood by the accompanying drawings and detailed descriptions, wherein:
Fig. 1 illustrates a schematic 3-D view of a first preferred embodiment of the ankle protecting device of the present invention;
Fig. 2 illustrates a schematic side view of a line II-II in Fig. 1 and shows the side shape of a foot bottom supporting plate;
Fig. 3 illustrates an application view of the ankle protecting device;
Fig. 4 illustrates another type of the foot bottom supporting plate and shows that the shape of the foot bottom supporting plate is to fit an arch; and
Fig. 5 illustrates a schematic 3-D view of a second preferred embodiment of the ankle protecting device of the present invention, wherein a foot sheath has a a top surface portion connecting a left side portion and a right side portion.

### DETAILED DESCRIPTION OF THE INVENTION

Following preferred embodiments and figures will be described in detail so as to achieve aforesaid objects.

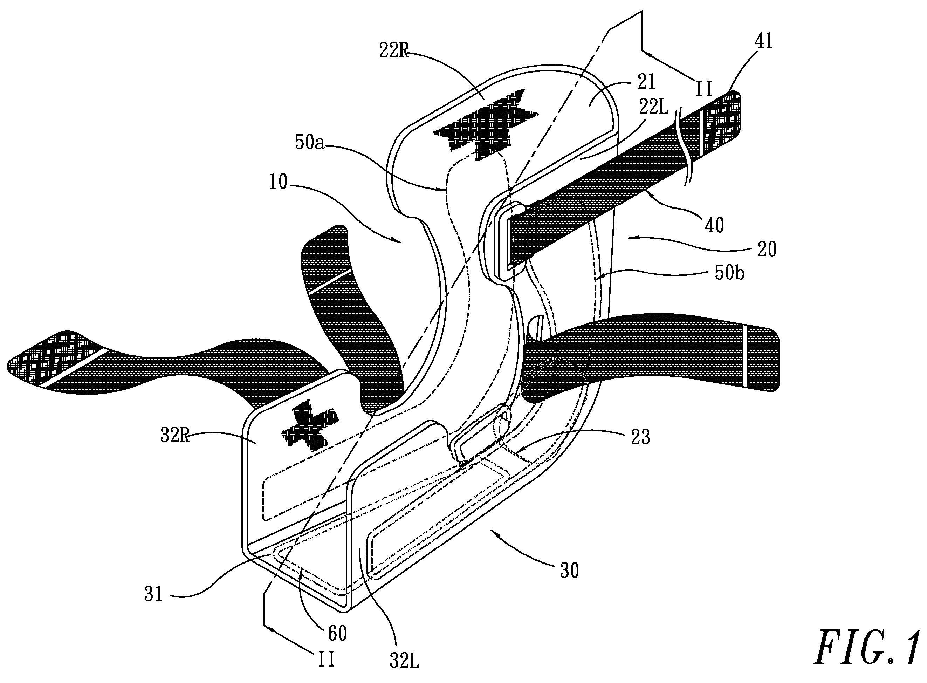

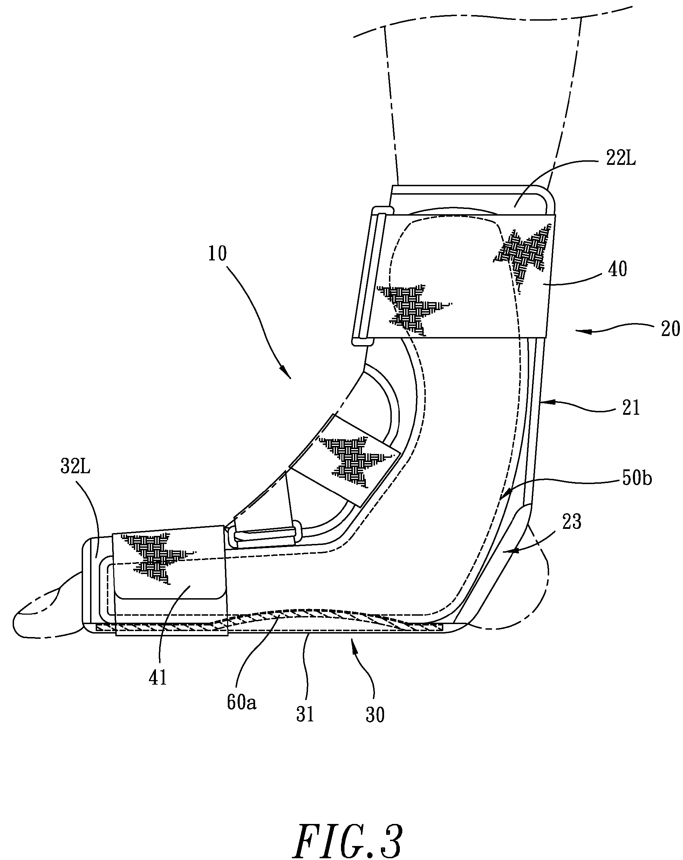

With reference to Fig. 1, which illustrates a schematic 3-D view of a first preferred embodiment of the ankle protecting device of the present invention. The ankle protecting device includes:
a wrapping sheath 10, which has a lower-leg sheath 20, a foot sheath 30 and a loincloth 40 that are integrated with thereof, the loincloth 40 constricts the wrapping sheath 10 on a sole, an ankle and a partial lower leg above the ankle and is adjustable for tightness thereof (referring to Fig. 3 as well);
an internal supporting plate 50a and an outer supporting plate 50b, which are fastened on the left and right sides of the wrapping sheath 10 respectively, the shapes of the internal supporting plate 50a and the outer supporting plate 50b are symmetrical to each other, the internal supporting plate 50a and the outer supporting plate 50b elongate downward to be over the ankle along the internal and outer sides of the lower leg and then to the two sides of the sole toward the top of the sole; and
a foot bottom supporting plate 60, which are fastened on the bottom 31 of the foot sheath 30.

A type of the wrapping sheath 10 in the first preferred embodiment is as shown in Fig. 1, both the lower-leg sheath 20 and the foot sheath 30 have three surfaces, the lower-leg sheath 20 has a back surface 21, a left side surface 22L and a right side surface 22R in order to wrap around the back side, the inner side and the outer side of the lower leg, the foot sheath 30 of the wrapping sheath 10 has a bottom portion 31, a left side portion 32L and a right side portion 32R in order to wrapped around the bottom portion, the inner side and the outer side of the sole, the connecting position of the back surface 21 of the lower-leg sheath 20 and the bottom portion 31 of the foot sheath 30 has a heel positioning hole 23 so as to expose the heel of a user.

The number of the loincloth 40 can be plural, and the loincloth 40 of the preferred embodiment is a flat ribbon fabric with a Velcro 41 thereon and winds around the peripheries of the lower-leg sheath 20 and the foot sheath 30. While the user wears the wrapping sheath 10 on the lower leg and the sole, the loincloths 40 are able to constrict the wrapping sheath 10 on the sole, the ankle and the partial lower leg above the ankle and adjust the tightness thereof according to the Velcros 41. Hence, a three-point support is formed by the foot bottom supporting plate 60, the internal supporting plate 50a and the outer supporting plate 50b in order to support the internal and outer sides of the lower leg, the ankle and the sole, and the angles of inner flipping, outer flipping, forward flipping, and backward flipping of the sole shall be restricted, so that the stability of the ankle is increased, the possibility of sprain is decreased, further, the effectiveness of oppression and preventing swelling are achieved as well (referring to Fig. 3).

Another type of the loincloth 40 is a flat ribbon fabric with plural buttons and button holes thereon and winds around the peripheries of the lower-leg sheath 20 and the foot sheath 30. The tightness of the loincloth 40 is adjustable through the fastening positions of the buttons and button holes. A third type of the loincloth 40 is a flat ribbon fabric with plural male/female buckles thereon and winds around the peripheries of the lower-leg sheath 20 and the foot sheath 30. The tightness of the loincloth 40 is adjustable through the fastening positions of the male buckles and female buckles.

One type of the flat ribbon fabric of the loincloth 40 is made of canvas that is not retractable but tough. By means of the fastening positions of the Velcros 41, the buttons and button holes and the male/female buckles, the tightness of the loincloth 40 is variable.

Another type of the flat ribbon fabric of the loincloth 40 is made of bandage that is retractable and elastic. By means of the fastening positions of the Velcros 41, the buttons and button holes and the male/female buckles, the tightness of the loincloth 40 is changeable.

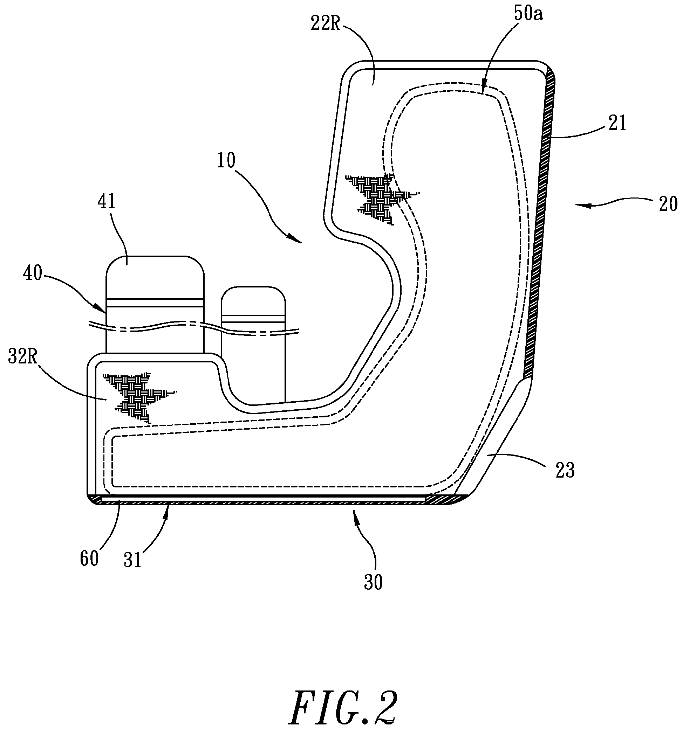

Another type of the wrapping sheath 10 is made of elastic fabric and cloth, the left and right sides of the wrapping sheath 10 and the bottom portion 31 of the foot sheath 30 have an interlining respectively, and the applied structure is consisted of a double-layer elastic fabric or a double-layer cloth sewed together. The internal supporting plate 50a, the outer supporting plate 50b and the foot bottom supporting plate 60 are made by aluminum or plastic. The foot bottom supporting plate 60 is disposed into the interlining of the bottom portion 31 of the foot sheath 30 (referring to Fig. 2). The internal supporting plate 50a and the outer supporting plate 50b are disposed into the interlinings of the left and right sides of the wrapping sheath 10. Hence, a three-point support is formed by the foot bottom supporting plate 60, the internal supporting plate 50a and the outer supporting plate 50b in order to support the internal and outer sides of the lower leg, the ankle and the sole, and the angles of inner flipping, outer flipping, forward flipping, and backward flipping of the sole shall be restricted, so that the stability of the ankle is increased, the possibility of sprain is decreased, further, the effectiveness of oppression and preventing swelling are achieved as well.

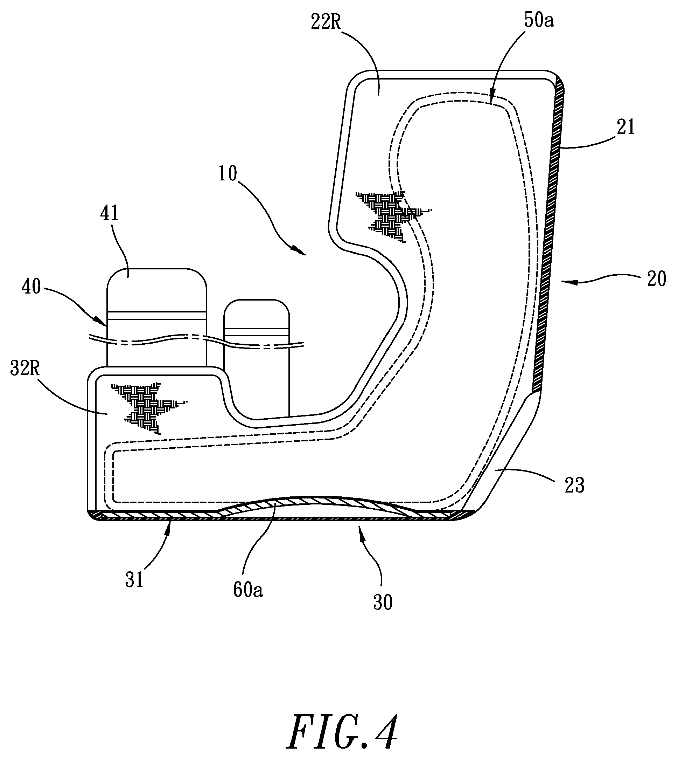

One type of the foot bottom supporting plate 60 is a planar element with a straight surface (referring to Fig. 2); another foot bottom supporting plate 60a is shaped to fit the arch of the sole in order to provide the comfort of the bottom of the sole and a support with ergonomics (referring to Fig. 4).

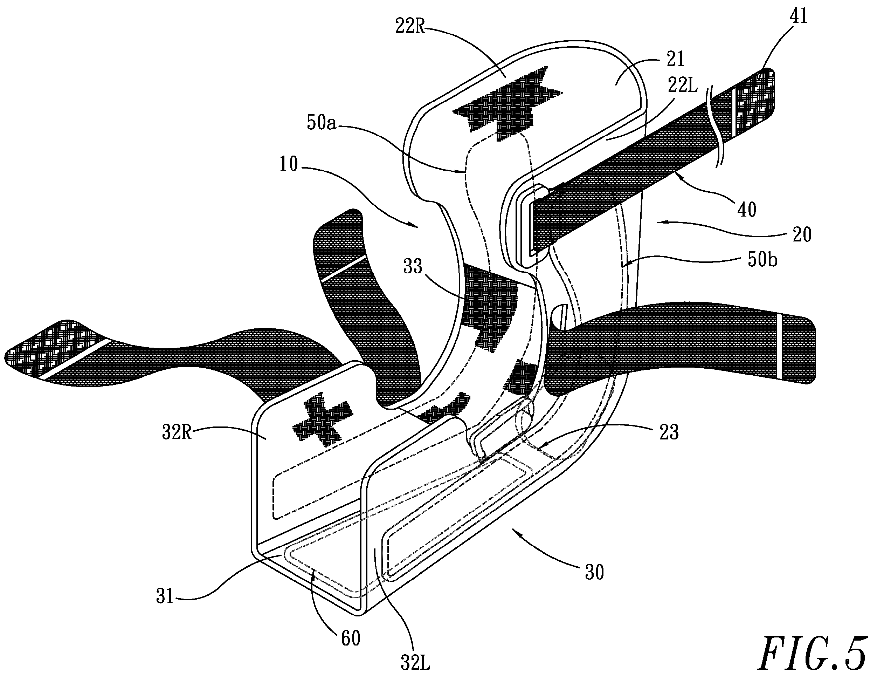

Another type of the wrapping sheath is as shown in Fig. 5, wherein the foot sheath 30 has a bottom portion 31, a left side portion 32L, a right side portion 32R, and a top surface portion 33 connecting the left side portion 32L and the right side portion 32R in order to wrapped around the bottom portion, the back portion, the inner side and the outer side of the sole, wherein the top surface portion 33 of the foot sheath 30 is made of an elastic fabric or a cloth, and a better feature is good permeability. Further, the top surface portion 33 of the foot sheath 30 is easily let the sole of the user be through but not loosed.

Although the invention has been disclosed and illustrated with reference to particular embodiments, the principles involved are susceptible for use in numerous other embodiments that will be apparent to persons skilled in the art. This invention is, therefore, to be limited only as indicated by the scope of the appended claims

## Claims

1. An ankle protecting device, comprising:
a wrapping sheath (10) having a lower-leg sheath (20), a foot sheath (30) and a loincloth (40) that are integrated with thereof, the loincloth (40) constricting the wrapping sheath (10) on a sole, an ankle and a lower leg and being adjustable for tightness thereof, the lower-leg sheath (20) of the wrapping sheath (10) has a back surface (21), a left side surface (22L) and a right side surface (22R) in order to wrap around the back side,
the inner side and the outer side of the lower leg, the foot sheath (30) of the wrapping sheath (10) having a bottom portion (31), a left side portion (32L) and a right side portion (32R) in order to wrap around the bottom portion, the inner side and the outer side of the sole, the connecting position of the back surface (21) of the lower-leg sheath (21) and the bottom portion (31) of the foot sheath (30) having a heel positioning hole (23), the number of the loincloth (40) can be plural, the loincloth (40) being a flat ribbon fabric with a Velcro® thereon and winding around the peripheries of the lower-leg sheath (20) and the foot sheath (30);
**characterised by** an internal supporting plate (50a) and an outer supporting plate (50b) fastened on the left and right sides of the wrapping sheath (10) respectively, the shapes of the internal supporting plate (50a) and the outer supporting plate (50b) being symmetrical to each other, the internal supporting plate (50a) and the outer supporting plate (50b) elongating to be over the ankle along the internal and outer sides of the lower leg and then to the two sides of the sole toward the top of the sole; and
a foot bottom supporting plate (60) fastened on the bottom (31) of the foot sheath (30).

2. The ankle protecting device according to claim 1, wherein the foot sheath (30) further comprises a top surface portion (33) connecting the left side portion (32L) and the right side portion (32R).

3. The ankle protecting device according to claim 1, wherein the wrapping sheath (10) is made of elastic fabric and cloth, so that the wrapping sheath (10) has retractility and elasticity, the left and right sides of the wrapping sheath (10) and the bottom portion (31) of the foot sheath (30) having an interlining respectively, the foot bottom supporting plate (60) being disposed into the interlining of the bottom portion (31) of the foot sheath (30), the internal supporting plate (50a) and the outer supporting plate (50b) being disposed into the interlinings of the left and right sides of the wrapping sheath (10).

4. The ankle protecting device according to claim 1, wherein the flat ribbon fabric of the loincloth (40) is made of canvas that is not retractable but tough.

5. The ankle protecting device according to claim 1, wherein the flat ribbon fabric of the loincloth (40) is made of bandage that is retractable and elastic.

6. The ankle protecting device according to claim 1, wherein the internal supporting plate (50a), the outer supporting plate (50b) and the foot bottom supporting plate (60) are made by aluminum or plastic.

7. The ankle protecting device according to claim 1, wherein the foot bottom supporting plate (60) is a planar element with a straight surface.

8. The ankle protecting device according to claim 1, wherein the foot bottom supporting plate (60) is shaped to fit the arch of the sole.

## Patentansprüche

1. Knöchelschutzeinrichtung, aufweisend:
einen Umhüllungswickel (10) mit einer unteren Fußumhüllung (20), einer Fußumhüllung (30) und einem Schurz (40), die damit integriert sind, wobei der Schurz (40) den Umhüllungswickel (10) auf einer Sohle, einen Knöchel und einen unteren Fuß beschränkt und für dessen Straffreiheit einstellbar ist, wobei die untere Fußumhüllung (20) des Umhüllungswickel (10) eine hintere Oberfläche (21), eine linke Seitenoberfläche (22L) und eine rechte Seitenoberfläche (22R) aufweist, um um die Rückseite gewickelt zu werden, wobei die Innenseite und die Außenseite des unteren Fußes, die Fußumhüllung (30) des Umhüllungswickel (10) einen unteren Bereich (31), einen linken Seitenbereich (32L) und einen rechten Seitenbereich (32R) aufweisen, um um den unteren Bereich gewickelt zu werden, wobei die Innenseite und die Außenseite des Sohle, die Verbindungsposition der hinteren Oberfläche (21) der unteren Fußumhüllung (21) und der untere Bereich (31) der Umhüllung (30) eine Absatzpositionierungsöffnung (23) aufweisen, wobei die Anzahl von Schürzen (40) mehrere betragen kann, wobei die Schürze (40) ein flaches Bandgewebe mit Velcro ® darauf ist, und eine Wicklung um das Äußere der unteren Fußumhüllung (20) und der Fußumhüllung (30) aufweist;
**gekennzeichnet durch**
eine innere Stützplatte (50a) und eine äußere Stützplatte (50b), die an der linken bzw. rechten Seite des Umhüllungswickel (10) angebracht sind, wobei die Formen der inneren Stützplatte (50a) und der äußeren Stützplatte (50b) symmetrisch zueinander sind, wobei die innere Stützplatte (50a) und die äußere Stützplatte (50b) über den Knöchel entlang der inneren Seite und äußeren Seite des unteren Fußes und dann zu den zwei Seiten der Sohle in Richtung der Oberseite der Sohle verlängert sind; und
eine Fußunterseitenstützplatte (60), die an der Unterseite (31) der Fußumhüllung (30) befestigt ist.

2. Knöchelschutzeinrichtung nach Anspruch 1, wobei die Fußumhüllung (30) ferner einen oberen Oberflächenbereichs (32) aufweist, der den linken Seitenbereich (32L) und den rechten Seitenbereich (32R) verbindet.

3. Knöchelschutzeinrichtung nach Anspruch 1, wobei der Umhüllungswickel (10) aus einem elastischen Gewebe oder Tuch gemacht ist, so dass der Umhüllungswickel (10) eine Rückziehbarkeit und eine Elastizität aufweist, wobei die linke und die rechte Seite des Umhüllungswickel (10) bzw. der untere Bereich (31) der Fußumhüllung (30) ein Zwischenfutter aufweisen, wobei die Fußunterseitenstützplatte (60) in das Zwischenfutter des unteren Bereichs (31) der Fußumhüllung (30) angeordnet ist, wobei die innere Stützplatte (50a) und die äußere Stützplatte (50b) in die Zwischenfutter der linken und der rechten Seite des Umhüllungswickel (10) angeordnet sind.

4. Knöchelschutzeinrichtung nach Anspruch 1, wobei das flache Bandgewebe des Schurz (40) aus Segeltuch gemacht ist, das nicht rückziehbar sondern robust ist.

5. Knöchelschutzeinrichtung nach Anspruch 1, wobei das flache Bandgewebe der Schürze (40) aus einer Bandage gemacht ist, die rückziehbar und elastisch ist.

6. Knöchelschutzeinrichtung nach Anspruch 1, wobei die innere Stützplatte (50a), die äußere Stützplatte (50b) und die Fußunterseitenstützplatte (60) durch Aluminium oder Kunststoff hergestellt sind.

7. Knöchelschutzeinrichtung nach Anspruch 1, wobei die Fußunterseitenstützplatte (60) ein ebenes Element mit einer geraden Oberfläche ist.

8. Knöchelschutzeinrichtung nach Anspruch 1, wobei die Fußunterseitenstützplatte (60) derart geformt ist, dass sie an den Bogen der Sohle passt.

## Revendications

1. Un dispositif de protection de la cheville comprenant:
une bande enveloppante (10) intégrant d'une part la partie inférieure de la jambe (20) et d'autre part le pied (30) ainsi qu'une gaine (40), cette gaine (40) permettant de maintenir la bande enveloppante (10) sur la plante du pied, la cheville et la partie inférieure de la jambe et de régler son étroitesse; la bande couvrant la partie inférieure de la jambe (20) de la bande enveloppante (10) présente une surface dorsale (21), une surface latérale gauche (22L) et une surface latérale droite (22R) permettant d'envelopper la partie arrière, le côté intérieur et le côté extérieur de la jambe inférieure; la bande du pied (30) de la bande enveloppante (10) dispose d'une partie basse (31), d'une partie latérale gauche (32L) et d'une partie latérale droite (32R) permettant d'envelopper la partie basse, le côté intérieur et le côté extérieur de la plante; le point de connexion de la surface arrière (21) de la partie inférieure de la jambe (21) et de la partie inférieure (31) de la bande du pied (30) présentent un trou au niveau du talon (23), la gaine (40) peut être constituée de plusieurs couches,
s'agissant (40) d'une bande de tissu plate recouverte de Velcro® et enroulée autour de la bande de la partie inférieure de la jambe (20) et du pied (30);
**caractérisé par**
une plaque de support interne (50a) et une plaque de support externe (50b) fixées respectivement sur le côté gauche et droit de la bande enveloppante (10), les formes de la plaque de support interne (50a) et externe (50b) étant symétriques l'une par rapport à l'autre, la plaque de support interne (50a) et la plaque de support externe (50b) s'allongeant le long de la cheville et le long des côtés extérieurs et intérieurs du bas de la jambe et ensuite vers le haut des deux côtés de la plante; et
une plaque de support (60) fixée sur le dessous (31) de la bande couvrant le pied (30).

2. Le dispositif de protection de la cheville décrit selon claim 1, où la bande du pied (30) comprend en outre une surface protectrice supérieure (33) reliant la partie latérale gauche (32L) et la partie latérale droite (32R).

3. Le dispositif de protection de la cheville selon claim 1, où la bande enveloppante est constituée (10) d'un tissu et d'un bandage élastiques, de sorte que la bande enveloppante (10) soit rétractable et élastique, où le côté gauche et droit de la bande enveloppante (10) ainsi que la partie inférieure (31) de la bande du pied (30) disposent respectivement d'une entretoile, la plaque de support inférieure (60) étant disposée dans l'entretoile de la partie inférieure (31) de la bande du pied (30), les plaques de support interne (50a) et externe (50b) étant disposées dans les entretoiles des côtés gauche et droit de la bande enveloppante (10).

4. Le dispositif de protection de la cheville selon claim 1, où la bande de tissu de la gaine (40) est constituée d'une toile non élastique et résistante.

5. Le dispositif de protection de la cheville selon claim 1, dans lequel le ruban plat de la gaine (40) est en bandage rétractable et élastique.

6. Le dispositif de protection de la cheville selon claim 1, où la plaque de support interne (50a), la plaque de support externe (50b) et la plaque de support inférieure (60) sont en aluminium ou en plastique.

7. Le dispositif de protection de la cheville selon claim 1, dans lequel la plaque de support inférieure du pied (60) est un élément plan avec une surface droite.

8. Le dispositif de protection de la cheville selon claim 1, où la plaque de support inférieure du pied (60) a une forme qui lui permet de s'adapter à la voûte plantaire.
